# EUROPÄISCHE PATENTSCHRIFT

(11) **EP 1 912 476 B1**
(45) Veröffentlichungstag und Bekanntmachung des Hinweises auf die Patenterteilung: **25.07.2012**
(21) Anmeldenummer: 07117687.9
(22) Anmeldetag: 02.10.2007
(51) Int. Cl.: H04R 25/00, G06F 19/00

(54) **Verfahren zum Trainieren auditiver Fähigkeiten**
Method for training auditive abilities
Procédé d'entraînement de capacités auditives

(30) Priorität: 09.10.2006 DE 102006047690
(43) Veröffentlichungstag der Anmeldung: 16.04.2008
(73) Patentinhaber: Siemens Audiologische Technik GmbH, 91058 Erlangen (DE)
(72) Erfinder: Chalupper, Josef, 85307 Paunzhausen (DE)
(74) Vertreter: Maier, Daniel Oliver

(56) Entgegenhaltungen:
- DE-U1- 20 118 619
- US-A1- 2006 029 912
- WRIGHT BA: "Why and how we study human learning on basic auditory tasks", AUDIOLOGY AND NEURO-OTOLOGY, KARGER, BASEL, CH, Bd. 6, 1. Januar 2001 (2001-01-01), Seiten 207-210, XP009145828, ISSN: 1420-3030

## Beschreibung

Die vorliegende Erfindung betrifft ein Verfahren zum Trainieren auditiver Fähigkeiten eines Hörgeräteträgers durch Darbieten eines Schallbeispiels mit dem Hörgerät, Aufnehmen und Auswerten der Reaktion des Hörgeräteträgers auf das Schallbeispiel und mehrfaches Wiederholen eines Trainingsschritts enthaltend die Schritte des Darbietens von unterschiedlichen Schallbeispielen sowie des Aufnehmens und Auswertens im Rahmen eines Trainings.

Hörgeräte sind tragbare Hörvorrichtungen, die zur Versorgung von Schwerhörenden dienen. Um den zahlreichen individuellen Bedürfnissen entgegenzukommen, werden unterschiedliche Bauformen von Hörgeräten wie Hinter-dem-Ohr-Hörgeräte (HdO), Indem-Ohr-Hörgeräte (IdO) und Concha-Hörgeräte bereitgestellt. Die beispielhaft aufgeführten Hörgeräte werden am Außenohr oder im Gehörgang getragen. Darüber hinaus stehen auf dem Markt aber auch Knochenleitungshörhilfen, implantierbare oder vibrotaktile Hörhilfen zur Verfügung. Dabei erfolgt die Stimulation des geschädigten Gehörs entweder mechanisch oder elektrisch.

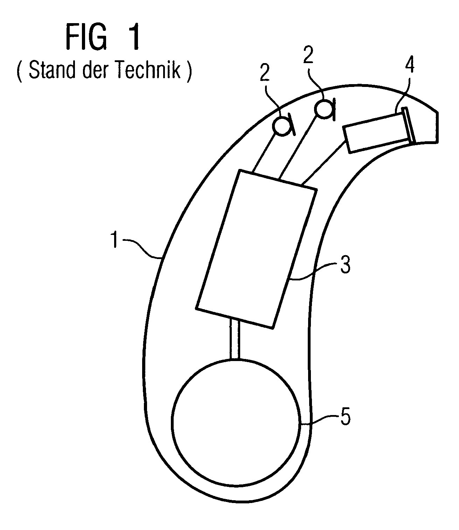

Hörgeräte besitzen prinzipiell als wesentliche Komponenten einen Eingangswandler, einen Verstärker und einen Ausgangswandler. Der Eingangswandler ist in der Regel ein Schallempfänger, z. B. ein Mikrofon, und/oder ein elektromagnetischer Empfänger, z. B. eine Induktionsspule. Der Ausgangswandler ist meist als elektroakustischer Wandler, z. B. Miniaturlautsprecher, oder als elektromechanischer Wandler, z. B. Knochenleitungshörer, realisiert. Der Verstärker ist üblicherweise in eine Signalverarbeitungseinheit integriert. Dieser prinzipielle Aufbau ist in FIG 1 am Beispiel eines Hinter-dem-Ohr-Hörgeräts dargestellt. In ein Hörgerätegehäuse 1 zum Tragen hinter dem Ohr sind ein oder mehrere Mikrofone 2 zur Aufnahme des Schalls aus der Umgebung eingebaut. Eine Signalverarbeitungseinheit 3, die ebenfalls in das Hörgerätegehäuse 1 integriert ist, verarbeitet die Mikrofonsignale und verstärkt sie. Das Ausgangssignal der Signalverarbeitungseinheit 3 wird an einen Lautsprecher bzw. Hörer 4 übertragen, der ein akustisches Signal ausgibt. Der Schall wird gegebenenfalls über einen Schallschlauch, der mit einer Otoplastik im Gehörgang fixiert ist, zum Trommelfell des Geräteträgers übertragen. Die Stromversorgung des Hörgeräts und insbesondere die der Signalverarbeitungseinheit 3 erfolgt durch eine ebenfalls ins Hörgerätegehäuse 1 integrierte Batterie 5.

Da aufgrund eines Hörverlusts viele Schalle nicht hörbar sind, "entwöhnt" sich die zentrale Verarbeitung im Gehirn des Schwerhörigen. Das bedeutet, dass durch ein Hörgerät diese Schalle zwar wieder hörbar sind, aber vom Schwerhörigen nicht interpretiert werden können, da die zentrale Verarbeitung im Gehirn verlernt hat, diese Schalle zu verarbeiten. Dies wird häufig auch als "Diskriminationsverlust", "auditorische Deprivation" oder "zentrale Verarbeitungsstörung" bezeichnet. Der betroffene Schwerhörige äußert dann typischerweise: "Ich höre, verstehe aber nichts!". Ein derartiges Phänomen wird häufig auch bei Kindern und in Verbindung mit Legasthenie beobachtet.

Da das Gehirn bis ins hohe Alter lernfähig bleibt, kann man durch ein gezieltes Hörtraining häufig derartige Verarbeitungsstörungen mindern oder komplett rehabilitieren. Das Problem ist, dass hierfür das Hörtraining möglichst oft (auch zuhause) durchgeführt werden muss und Apparaturen zur Darbietung von Schallen benötigt werden (z. B. Stereoanlage). An der Verfügbarkeit und Bedienung derartiger Apparaturen scheitert meist das Durchführen eines Hörtrainings in kurzen Zeitabständen.

Zentrale Verarbeitungsstörungen sind häufig auch ein Grund dafür, dass Hörgeräteträger die technischen Möglichkeiten moderner Hörgeräte nicht in vollem Umfang nutzen können. So ist beispielsweise der Nutzen von Richtmikrofonen für Normalhörende in der Regel größer als für Schwerhörige.

Bislang wird das Hörtraining nahezu ausschließlich bei Akustikern oder anderen entsprechend ausgebildeten Personen durchgeführt, die über geeignete Apparaturen verfügen. Das Hörtraining ist deshalb mit regelmäßigen Sitzungen bei diesem Hörtrainer verbunden. Das ist einerseits für Kinder und ältere Menschen sehr aufwändig, weshalb dieses Angebot nur selten angenommen wird. Außerdem ist ein derartiges Training wenig effektiv, da außerhalb der Sitzungen das Gehör nicht trainiert werden kann.

In der nachveröffentlichten Druckschrift DE 10 2005 034 381 ist ein Verfahren zum Darbieten von Funktionsmöglichkeiten eines Hörgerätesystems beschrieben. Die einzelnen Verfahrensschritte zum Darbieten der Funktionsmöglichkeiten des Hörgerätesystems können in ein Spielprogramm integriert sein. So kann beispielsweise durch Hörrätsel gezielt das Hören mit dem Hörgerät trainiert werden. Außerdem kann durch geeignete Soundbeispiele das räumliche Hören trainiert werden.

Hinsichtlich des Lernens, dargebotene Schalle richtig zu interpretieren, wird zusätzlich auf die folgenden beiden Artikel verwiesen: Wright, B.A. (2001) "Why and how we study human learning on basic auditory tasks." Audiology and Neuro-Otology 6, 207-210 und Wright, B.A. and Fitzgerald, M.B. (2001), "Different patterns of human disrimination learning for two interaural cues to sound-source location, "PROCEEDINGS OF THE NATIONAL ACADEMY OF SCIENCES Vol. 98, Issue 21, 12307-12312.

Aus der Druckschrift DE 103 93 463 T5 ist eine Trainingsvorrichtung bekannt, die ein geräuschvocodiertes Sprachschallsignal darbietet, eine Antwort von einem Trainierenden empfängt und ein Ergebnis ausgibt, welches anzeigt, ob die Antwort korrekt oder falsch ist. Mehrere Wörter oder Sätze können wiederholt trainiert werden.

Darüber hinaus offenbart die Druckschrift DE 201 18 619 U1 ein Differenzton-Trainingsgerät für Personen mit störenden Ohrgeräuschen. Auf eine Tonanfrage des Geräts muss der Nutzer bewusst und aktiv mit einer Tastenquittung reagieren. Durch die zwingend erforderliche Rückmeldung des Nutzers auf die angebotenen Tonpaare wird ein Frage-Antwort-Zyklus realisiert, der nach vielfacher Ausführung einen Lerneffekt bewirkt.

Aus der Druckschrift US 2006/0029912 A1 ist ein System zur Gehörrehabilitation bekannt. Das System weist ein lokales Gerät auf, das ein Hörgeschädigter tragen kann. Mit dem System ist ein Training möglich, über das ein Protokoll geführt wird. Das Training wird angehalten, wenn das Trainingsergebnis ausreichend ist oder wenn eine ausreichende Anzahl an Trainingsschritten durchgeführt wurde. Mit statistischen Methoden kann ein erwarteter Fortschritt mit einem aktuellen Fortschritt verglichen werden.

Die Aufgabe der vorliegenden Erfindung besteht darin, das Hörtraining für einen Hörgeräteträger effektiver zu gestalten.

Erfindungsgemäß wird diese Aufgabe gelöst durch ein Verfahren zum Trainieren auditiver Fähigkeiten eines Hörgeräteträgers durch folgende von einem Hörgerät durchgeführten Schritte: Darbieten eines Schallbeispiels mit dem Hörgerät, Aufnehmen und Auswerten der Reaktion des Hörgeräteträgers auf das Schallbeispiel und mehrfaches Wiederholen eines Trainingsschritts enthaltend die Schritte des Darbietens von unterschiedlichen Schallbeispielen sowie des Aufnehmens und Auswertens im Rahmen eines Trainings und Rückmelden eines Fortschritts des Trainingserfolgs des Trainings an den Hörgeräteträger.

Durch die erfindungsgemäße Möglichkeit, ein Hörtraining mit dem eigenen Hörgerät durchzuführen und den Trainingserfolg mitgeteilt zu bekommen, ist eine Verbesserung und Überprüfung der Hör- und Kommunikationsfähigkeit für alle Hörgeräteträger möglich, auch für die, die an regelmäßigen Hörtrainingssitzungen nicht teilnehmen können. Darüber hinaus ergibt sich unter Umständen der Vorteil, dass technische Möglichkeiten moderner Hörsysteme nun besser oder voll genutzt werden können. So können beispielsweise Richtmikrofone oder eine Verstärkung hoher Frequenzen für erfolgreich trainierte Hörgeräteträger einen deutlichen Nutzen bringen.

Das erfindungsgemäße Hörtraining mit dem Hörgerät kann darüber hinaus zu einer Destigmatisierung des Hörgeräts beitragen, da es auch für die Therapie von zentralen Verarbeitungsstörungen und Legasthenie verwendet werden kann. Außerdem können mit dem vorgestellten Verfahren bereits vorhandene Hörtrainingsmethoden unterstützt werden.

Vorzugsweise wird im Rahmen des erfindungsgemäßen Verfahrens beim Rückmelden eine Veränderung des Trainingserfolgs gegenüber dem Start des Trainings gemeldet. Damit erhält der Trainierende einen Anhaltspunkt über den subjektiven Fortschritt seines Trainings.

Alternativ oder zusätzlich kann bei dem Rückmelden ein Abstand des aktuellen Trainingsstatus gegenüber einem Normahörenden gemeldet werden. Hierdurch kann der Trainierende in etwa einschätzen, wie weit das Training bereits fortgeschritten ist und wie lange es gegebenenfalls noch dauern kann.

Entsprechend einer speziellen Ausführungsform der vorliegenden Erfindung kann das Training für eine Verbesserung der Unterscheidbarkeit von Schallen eingesetzt werden. Hierzu ist es beispielsweise von Vorteil, wenn bei jedem Trainingsschritt zwei Schalle dargeboten werden, die sich in den hohen Frequenzen unterscheiden, und beim Auswerten eine Diskriminationsschwelle bestimmt wird. Damit kann objektiv die Unterscheidungsfähigkeit von Schallen ermittelt und dem Hörgeräteträger entsprechende Rückkopplungen gegeben werden.

Gemäß einem weiteren Ausführungsbeispiel kann das Training auf eine Verbesserung des räumlichen Hörens zielen. Dieses Training lässt sich beispielsweise so gestalten, dass bei jedem Trainingsschritt jeweils ein erster Schall, der für das linke und rechte Ohr gleich ist, jedoch unterschiedliche Pegel aufweist, und anschließend ein zweiter Schall, der für beide Ohren gleich ist und auch gleiche Pegel aufweist, dargeboten wird. Auf diese Weise ist es möglich, eine interaurale Pegelunterschiedsschwelle zu bestimmen, die als Richtgröße für das räumliche Hören herangezogen werden kann.

Darüber hinaus ist entsprechend einem weiteren Ausführungsbeispiel vorgesehen, das zeitliche Auflösen von Schallen durch den Hörgeräteträger zu verbessern. Dies erfolgt vorzugsweise dadurch, dass bei jedem Trainingsschritt jeweils ein Schallpaar bestehend aus einem durchgehenden ersten Ton und einem unterbrochenen zweiten Ton mit in der Länge vorgebbarer Lücke dargeboten wird. Auf diese Weise kann die für die Psychoakustik notwendige Lückendetektionsschwelle gewonnen werden.

Die vorliegende Erfindung ist anhand der beigefügten Zeichnungen näher erläutert, in denen zeigen:
- FIG 1: eine Skizze zum schematischen Aufbau eines Hörge- räts und
- FIG 2: ein Flussdiagramm zu einem Ausführungsbeispiel des erfindungsgemäßen Trainingsverfahrens.

Die nachfolgend näher geschilderten Ausführungsbeispiele stellen bevorzugte Ausführungsformen der vorliegenden Erfindung dar.

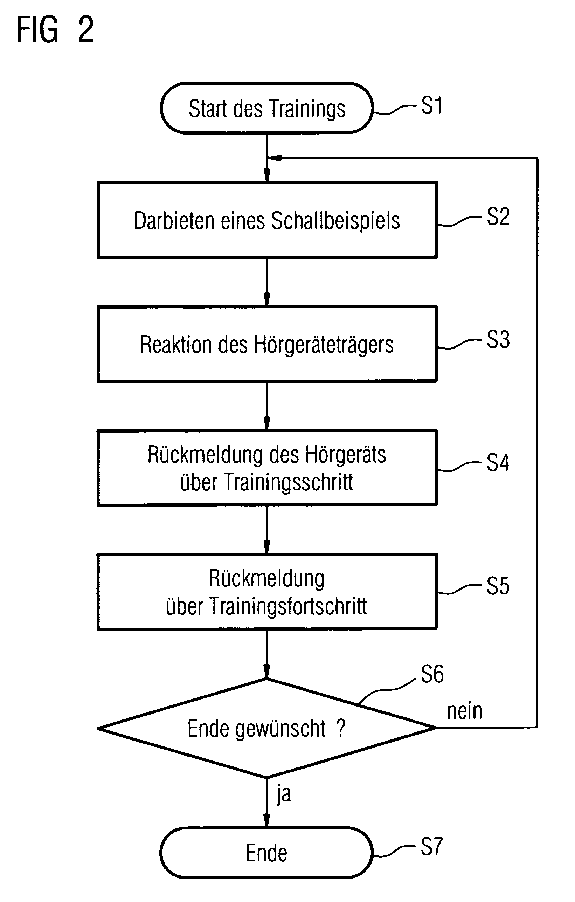

Ein bedeutender Aspekt der vorliegenden Erfindung besteht darin, dass das Hörtraining vom Benutzer alleine mit dem Hörgerät und eventuell mit einer zugehörigen Fernbedienung durchgeführt werden kann. Auf dem Hörgerät sind verschiedene Übungen gespeichert, die per Knopfdruck gestartet werden können, wie dies in FIG 2 mit Schritt S1 symbolisch angedeutet ist. Ein Start kann aber auch beispielsweise automatisch zu bestimmten Zeiten oder in bestimmten Intervallen erfolgen.

Nach dem Start beginnt das Training in einem ersten Trainingsschritt. Dabei wird dem Trainierenden ein entsprechendes Schallbeispiel dargeboten (Schritt S2). Der Trainierende beziehungsweise Hörgeräteträger gibt per Knopfdruck auf dem Hörgerät beziehungsweise der Fernbedienung seine Entscheidung bekannt (Schritt S3) und das Hörgerät meldet visuell oder akustisch zurück (Schritt S4), ob die Entscheidung richtig oder falsch war. Damit ist prinzipiell ein Trainingsschritt beendet.

Dem Hörgeräteträger wird darüber hinaus akustisch oder visuell rückgemeldet (Schritt S5), welche Fortschritte er im Training macht. Hierzu wird dem Hörgeräteträger beispielsweise die Verbesserung gegenüber dem Start des Hörtrainings und/oder der letzten Übung mitgeteilt. Optional kann dem Hörgeräteträger auch der Abstand seines Trainingszustands zu einem mittleren Normalhörenden mitgeteilt werden. Die Rückmeldung des Fortschritts kann wie in FIG 2 angedeutet nach jedem Trainingsschritt S2 bis S4 oder am Ende des Trainings vor Schritt S7 erfolgen.

Wünscht der Hörgeräteträger in Schritt S6 das Ende des Trainings, so wird dieses gemäß Schritt S7 beendet. Anderenfalls wird mit Schritt S2 ein neuer Trainingsschritt eingeleitet.

Der Hörgeräteakustiker oder Hörtrainer kann per Anpass-Software die geeigneten Übungen und die Zeiten/Intervalle auswählen. Über die Anpass-Software hat der Hörgeräteakustiker auch die Möglichkeit, zusätzliche Materialien (Fragebögen, Übungsanleitungen) auszudrucken und den Erfolg des Hörtrainings zu überwachen und auszudrucken. In der oben bereits angedeuteten Fachliteratur von Wright sind die benötigten Methoden und die zu erwartenden Lerneffekte beschrieben.

Im Folgenden ist eine kleine Auswahl möglicher Übungen dargestellt. Mit einem gekoppelten Hörsystem, welches die richtige Antwort rückmelden kann, können prinzipiell alle in der Literatur bekannten psychoakustischen Messmethoden (auch mit dynamischen Stimuli, z. B. dynamische ILD (= interaural level differenz), zum Hörtraining im Hörgerät verwendet werden.

### Beispiel 1:

Training zur Verbesserung der Unterscheidbarkeit von Schallen (Diskrimination). Dieses Training stellt prinzipiell eine monaurale Aufgabe dar.
1. Der Benutzer startet das Hörtraining per Fernbedienung (langer Druck auf Programmwechseltaste oder speziellen Knopf) oder beispielsweise durch den Programmschalter am Gerät.
2. Das Hörgerät spielt hintereinander zwei Schalle ab, deren Spektrum bei hohen Frequenzen leicht unterschiedlich sind. Hierfür wird ein im Hörgerät eingebauter Synthesizer oder Tongenerator verwendet.
3. Der Benutzer meldet zurück, ob die Schalle gleich sind oder unterschiedlich. Hierfür verwendet er zwei Tasten auf der Fernbedienung oder ein einmaliges beziehungsweise zweimaliges Drücken des Programmschalters.
4. Das Hörgerät meldet zurück, ob die Entscheidung richtig war. Durch diese Rückkopplung vollzieht der Hörgeräteträger einen Lernschritt.
5. Ein nächstes Schallpaar mit größerem oder kleinerem Unterschied als zuvor wird abgespielt usw.
6. Durch eine geeignete Änderung der Größe der Unterschiede von Schallpaar zu Schallpaar kann die Diskriminationsschwelle (ab der ein Unterschied hörbar ist) bestimmt und mit Daten für Normalhörende verglichen werden.

### Beispiel 2:

Training zur Verbesserung des räumlichen Hörens (Unterscheidbarkeit interauraler Pegeldifferenzen). Dies entspricht einer binauralen Aufgabe, d. h. es wird eine Kopplung beider Geräte benötigt.
1. Der Benutzer startet das Hörtraining per Fernbedienung oder durch Programmschalter am Gerät wie oben.
2. Das Hörgerät spielt ein Schallpaar ab:
   Schall 1: links und rechts der gleiche Schall (gleiches Spektrum und gleiche Phasenlage), jedoch mit links und rechts unterschiedlichen Pegeln. Bekanntermaßen bestimmen nämlich die interauralen Pegelunterschiede die links-rechts-Ortung. Ist der Pegel rechts größer als links, wird das Geräusch als von rechts kommend wahrgenommen.
   Schall 2: links und rechts gleicher Schall; auch der Pegel ist gleich.
3. Der Benutzer bestimmt, welcher Schall als "weiter rechts" wahrgenommen wird.
4. Das Hörgerät meldet zurück, ob die Entscheidung richtig war. Anhand dieser Rückmeldung lernt der Hörgeräteträger.
5. Ein nächstes Schallpaar mit größerem oder kleinerem Pegelunterschied als zuvor wird abgespielt usw.
6. Durch eine geeignete Änderung der Größe der Unterschiede von Schallpaar zu Schallpaar kann die interaurale Pegelunterschiedsschwelle bestimmt und mit Daten für Normalhörende verglichen werden.

### Beispiel 3:

Training zur Verbesserung der Zeitauflösung. Auch dies stellt eine monaurale Aufgabe dar.
1. Der Benutzer startet das Hörtraining per Fernbedienung oder durch den Programmschalter am Gerät wie in den obigen Bespielen.
2. Das Hörgerät spielt ein Schallpaar ab:
   Schall 1: durchgehender Ton;
   Schall 2: Ton mit kurzer Pause ("Lücke"). Hierzu ist zu wissen, dass bei genügend kurzen Pausen das Gehör einen durchgehenden Ton wahrnimmt.
3. Der Benutzer bestimmt, welcher Schall eine Lücke hat.
4. Das Hörgerät meldet zurück, ob die Entscheidung richtig war. Der Hörgeräteträger lernt hierdurch.
5. Ein nächstes Schallpaar mit größerer oder kleinerer Lücke als zuvor wird abgespielt usw.
6. Durch geeignete Änderung der Größe der Unterschiede von Schallpaar zu Schallpaar kann die Lückendetektionsschwelle bestimmt und mit Daten für Normalhörende verglichen werden.

Jedes der oben aufgeführten Trainingsbeispiele stellt ein Training dar, das der Hörgeräteträger selbst zu jeder Zeit an seinem Hörgerät durchführen kann. Somit kann das Training ohne Aufwand häufig durchgeführt werden, so dass relativ rasch ein gewünschter Trainingserfolg erzielt werden kann.

## Patentansprüche

1. Verfahren zum Trainieren auditiver Fähigkeiten eines Hörgeräteträgers durch folgende von einem Hörgerät durchgeführten Schritte:
- Darbieten eines Schallbeispiels (S2),
- Aufnehmen und Auswerten (S4) der Reaktion (S3) des Hörgeräteträgers auf das Schallbeispiel und
- mehrfaches Wiederholen des Trainingsschritts enthaltend die Schritte des Darbietens von unterschiedlichen Schallbeispielen sowie des Aufnehmens und Auswertens im Rahmen eines Trainings,
**gekennzeichnet durch**
- Rückmelden eines Fortschritts (S5) des Trainingserfolgs des Trainings an den Hörgeräteträger.

2. Verfahren nach Anspruch 1, wobei bei dem Rückmelden (S5) eine Veränderung des Trainingserfolgs gegenüber dem Start des Trainings gemeldet wird.

3. Verfahren nach Anspruch 1 oder 2, wobei bei dem Rückmelden (S5) ein Abstand des aktuellen Trainingsstatus gegenüber einem Normalhörenden gemeldet wird.

4. Verfahren nach einem der vorhergehenden Ansprüche, wobei bei jedem Trainingsschritt zwei Schalle dargeboten werden, die sich in den hohen Frequenzen unterscheiden und beim Auswerten eine Diskriminationsschwelle bestimmt wird.

5. Verfahren nach einem der Ansprüche 1 bis 3, wobei bei jedem Trainingsschritt jeweils ein erster Schall, der für das linke und rechte Ohr gleich ist jedoch unterschiedliche Pegel aufweist, und anschließend ein zweiter Schall, der für beide Ohren gleich ist und auch gleiche Pegel aufweist, dargeboten wird.

6. Verfahren nach einem der Ansprüche 1 bis 3, wobei bei jedem Trainingsschritt jeweils ein Schallpaar bestehend aus einem durchgehenden ersten Ton und einem unterbrochenen zweiten Ton mit in der Länge vorgebbarer Lücke dargeboten wird.

## Claims

1. Method for training auditory skills of a hearing aid wearer by means of the following steps performed by a hearing aid:
- presenting a sound sample (S2),
- recording and analysing (S4) the response (S3) of the hearing aid wearer to the sound sample, and
- repeating the training step multiple times, including the steps of presenting different sound samples as well as recording and analysing within the framework of a training session,
**characterised by**
- feeding back progress (S5) in the training success of the training to the hearing aid wearer.

2. Method according to claim 1, wherein a change in the training success compared with the start of the training is reported in the feedback (S5).

3. Method according to claim 1 or 2, wherein a distance in the current training status compared with a person with normal hearing is reported in the feedback (S5).

4. Method according to one of the preceding claims, wherein in each training step two sounds which differ in the high frequencies are presented and during the analysis a discrimination threshold is determined.

5. Method according to one of claims 1 to 3, wherein in each training step a first sound which is identical for the left and right ear, but has different levels, is presented in each case, and then a second sound which is identical for both ears and also has identical levels is presented.

6. Method according to one of claims 1 to 3, wherein in each training step a sound pair is presented consisting in each case of a continuous first tone and an interrupted second tone with a gap that is predefinable in terms of its length.

## Revendications

1. Procédé d'entraînement de capacités auditives d'un porteur d'une prothèse auditive par les stades suivants effectués par une prothèse auditive :
- l'offre d'un exemple ( S2 ) de son,
- l'enregistrement et l'exploitation (S4) de la réaction ( S3 ) du porteur de la prothèse auditive à l'exemple de son et
- la répétition multiple du stade d'entraînement comportant les stades de l'offre d'exemples de son différents, ainsi que de l'enregistrement et de l'exploitation dans le cadre d'un entraînement,
**caractérisé par**
- l'annonce en retour d'un progrès (S5) du succès de l'entraînement au porteur de la prothèse auditive.

2. Procédé suivant la revendication 1, dans lequel, lors de l'annonce (S5) en retour, on annonce une modification du succès de l'entraînement par rapport au début de l'entraînement.

3. Procédé suivant la revendication 1 ou 2, dans lequel, lors de l'annonce (S5) en retour, on annonce un écart de l'état présent de l'entraînement par rapport à une écoute normale.

4. Procédé suivant l'une des revendications précédentes, dans lequel, à chaque stade de l'entraînement, on offre deux sons, qui se distinguent dans les hautes fréquences, et, à l'exploitation, on détermine un seuil de discrimination.

5. Procédé suivant l'une des revendications 1 à 3, dans lequel, pour chaque stade d'entraînement, on offre respectivement un premier son, qui est le même pour l'oreille gauche et l'oreille droite, mais en ayant des niveaux différents, et ensuite un deuxième son, qui est le même pour les deux oreilles et qui a aussi le même niveau.

6. Procédé suivant l'une des revendications 1 à 3, dans lequel, pour chaque stade d'entraînement, on offre respectivement une paire de sons constitués d'un premier ton continu et d'un deuxième ton interrompu ayant des lacunes de longueur pouvant être prescrites.
